# EUROPÄISCHE PATENTSCHRIFT

(11) **EP 0 490 384 B1**
(45) Veröffentlichungstag und Bekanntmachung des Hinweises auf die Patenterteilung: **19.07.1995**
(21) Anmeldenummer: 91121363.5
(22) Anmeldetag: 12.12.1991
(51) Int. Cl.: C12P 21/00, A61K 38/00

(54) **Verfahren zur Herstellung eines pasteurisierten und eisenfreien Human-Transferrins**
Process for the production of pasteurized and iron-free human transferrin
Procédé de production de transferrine humain pasteurisé et non ferrugineux

(30) Priorität: 13.12.1990 DE 4039721
(43) Veröffentlichungstag der Anmeldung: 17.06.1992
(73) Patentinhaber: BEHRINGWERKE Aktiengesellschaft, 35001 Marburg (DE)
(72) Erfinder: Haupt, Heinz, W-3550 Marburg (DE)

(56) Entgegenhaltungen:
- EP-A- 0 035 204
- US-A- 4 841 026

## Beschreibung

Die Erfindung betrifft ein verfahren zur Herstellung eines pasteurisierten und eisenfreien Human-Transferrins sowie seine Verwendung.

Transferrin ist das Transportprotein für Eisen im Blutplasma der Wirbeltiere einschließlich des Menschen.

Human-Transferrin wird hauptsächlich als Wachstumsfaktor für Zellkulturen, z.B. zur Vermehrung von Säugerzellen in der Gentechnik und als Therapeutikum in den seltenen Fällen von Atransferrinämie verwendet.

Für die Herstellung von Human-Transferrin aus Serum oder Plasma sind eine Reihe von verfahren beschrieben worden, die sich der verschiedensten Fraktionierungsprizipien bedienen und im technischen Maßstab durchführbar sind, beispielsweise durch stufenweise Fällungen mit Hilfe von Äthanol-Wasser-Gemischen in der Kälte, wobei Transferrin in kristalliner Form erhalten wird, durch Fällung mit Äthylalkohol in Gegenwart von Calcium- und Zink-Ionen sowie durch Adsorption von Verunreinigungen an Carboxymethyl- und DEAE-Zellulose, wobei therapeutisch anwendbares, pasteurisiertes, eisenabgesättigtes Transferrin erhalten wird, durch fraktionierte Aussalzung mit Hilfe von Ammoniumsulfat sowie Adsorption von Verunreinigungen an Aluminiumhydroxid in wäßrigem Milieu, mit Hilfe eines kombinierten Rivanol-Alkohol-Verfahrens, wobei Human-Transferrin mit einem Reinheitsgrad von mindestens 85 % isoliert wurde, das sich anschließend ohne Schwierigkeiten in reiner Form mit guten Ausbeuten kristallisieren ließ, sowie mittels einer Kombination von Fällungsschritten mit Rivanol und Ammoniumsulfat sowie der Adsorption von Verunreinigungen an Aluminiumhydroxid.

Neben diesen für die technische Isolierung von Human-Transferrin geeigneten Reinigungsverfahren sind in der Literatur eine Reihe weiterer Methoden beschrieben worden, die auf die Herstellung kleiner Transferrin-Mengen zu Forschungszwecken zugeschnitten sind.

Obwohl Human-Transferrin wie alle Plasmapräparate heute ausschließlich aus HBsAg-negativem und Anti-HIV-negativem Plasma hergestellt wird und aufgrund des Herstellungsverfahren und nach jahrelanger klinischer Anwendung im Hinblick auf die Übertragung von viruserkrankungen in die Gruppe der "low-risk" Präparate einzustufen ist, kann ein Restrisiko der Viruskontamination nicht ausgeschlossen werden. Es erschien daher wünschenswert, neben der bereits schon bestehenden Viruselimination einen zusätzlichen effizienten virusinaktivierenden Schritt mit dem Herstellungsverfahren zu kombinieren, um den derzeit höchstmöglichen Sicherheitsstandard zu erreichen.

Es sind im wesentlichen drei Verfahren für die Sterilisation von Plasmaproteinen bekannt: die Hitzebehandlung in flüssigem oder trockenem Zustand; die kombinierte Behandlung mit β-Propiolacton und UV-Licht-Bestrahlung und die Behandlung mit Äther oder Tri-n-butylphosphat in Kombination mit Detergenzien.

Eisenabgesättigtes Transferrin kann 10 Stunden bei 60°C in Lösung pasteurisiert werden, während Apotransferrin unter diesen Bedingungen denaturiert wird.

Alle diese Verfahren haben im Hinblick auf die Herstellung eines eisenfreien Transferrin-Präparates den Nachteil, daß zugesetztes oder natürlicherweise an Transferrin gebundenes Eisen im Anschluß an die Sterilisation in einem separaten Schritt mit Hilfe eines Komplexbildners (in der Regel EDTA) abgetrennt werden muß.

Aufgabe der vorliegenden Erfindung ist ein Verfahren bereitzustellen, das die Abtrennung und Komplexierung des transferrin-gebundenen Eisens und die Stabilisierung des gebildeten Apotransferrins während des 10stündigen Erhitzungsprozesses bei 60°C gleichzeitig ermöglicht.

Es war bekannt, Komplexbildner als Stabilisatoren bei der Pasteurisierung von C1-Inaktivator, alpha₁-Antitrypsin oder bestimmten Gerinnungsfaktoren zu verwenden, doch war keineswegs vorherzusehen, daß auch die biologische Aktivität des Apotransferrins bei der Sterilisation in Gegenwart solcher Verbindungen erhalten bleiben würden.

Gegenstand der Erfindung ist ein Verfahren zur Pasteurisierung von Human-Transferrin, dadurch gekennzeichnet, daß eine Lösung von Human-Transferrin, die einen Komplexbildner enthält, pasteurisiert wird.

Zu diesem Zweck kann man eine nach bekannten Methoden vorgereinigte 10-100 g, vorzugseise 40-60 g Transferrin pro 1 enthaltende Lösung mit soviel eines Komplexbildners, vorzugsweise einem löslichen Citrat oder einem Salz der Ethylendiamin -tetraessigsäure (EDTA), daß eine Konzentration von mindestens 0,4 mol/l erhalten wird, bevorzugt 1,2 M Natriumcitrat oder 0,5 M EDTA, versetzen, den pH-Wert vorzugsweise mit Zitronensäure auf 7-8 einstellen und den Ansatz 10 Stunden bei 60°C halten.

Während unter diesen Bedingungen das gebildete Apotransferrin vor der thermischen Inaktivierung geschützt ist, werden bei diesem Prozeß experimentell zugesetzte Modellviren schon nach kurzen Inkubationszeiten inaktiviert.

Zum Beweis wurden Proben von Human-Transferrin (5 % in 1,2 M Citrat pH 7,5) im Verhältnis 10:1 mit HSV-1, Poliovirus Typ 1 oder Vacciniavirus versetzt. Diese Proben wurden in einem temperaturkontrollierten Wasserbad bei 60°C in Zeitintervallen bis zu 10 Stunden erhitzt. Proben wurden nach 30 Minuten, 1, 2, 4, 6, 8 und 10 Stunden entnommen und sofort auf ihren Virusgehalt titriert. Die Titration von Herpes simplex Virus und Vacciniavirus erfolgte auf Verozellen, die von Poliovirus auf HEP-2-Zellen bei jeweils acht Parallelkulturen pro Virusverdünnungstufe. Wurde bei einer solchen Titration (untere Nachweisgrenze 1,5 log ID₅₀/ml) kein infektiöses Virus nachgewiesen, so wurde im Doppelansatz ein Volumen entsprechend 1 ml Originalprobe in einzelnen Kulturflaschen geprüft. Blieben alle Prüfkulturen negativ, so wurde der Virustiter mit ≦ 10 ID₅₀/ml angegeben. Die Titerberechnungen erfolgten nach Reed und Muench.

### Ergebnisse: (Alle Titerangaben als log 10 ID₅₀/ml)

HSV-1: Virustiter im Transferrin-Virusgemisch vor der Inaktivierung 6,4. Kein infektiöses Virus nachweisbar, ab 1 Stunde Inaktivierungszeit.
Poliovirus: Virustiter im Transferrin-Virusgemisch 5,7. Kein infektiöses Virus nachweisbar ab 30 Minuten Inaktivierungszeit.
Vacciniavirus: Virustiter im Transferrin-Virusgemisch 6,5. Kein infektiöses Restvirus nachweisbar ab 2 Stunden Inaktivierungszeit.

Die kurzen Inaktivierungszeiten, die zur vollständigen Inaktivierung der drei Modellviren ausreichte, zeigen, daß durch die Pasteurisierung von Transferrin eine sehr effiziente Inaktivierung von unbehüllten, behüllten und von komplex aufgebauten Viren möglich ist. HSV-1 kann als Modell für die große Gruppe der Viren, die von einer lipidhaltigen Hülle umgeben sind, angesehen werden und zeichnet sich in dieser Gruppe durch eine relativ hohe Stabilität aus. Retroviren, wie die HIV-Viren lassen sich in allen bisher untersuchten Fällen schneller inaktivieren als HSV. Poliovirus und Vacciniavirus stehen für ungehüllte Viren, deren erheblich höhere Hitzestabilität bekannt ist. Auch diese beiden Modellviren werden trotz ihrer hohen Stabilität unerwartet schnell inaktiviert. Die Pasteurisierung von Transferrin ist somit eine sehr effiziente Methode zur Inaktivierung von behüllten und unbehüllten Viren verschiedener Art.

Die Erhitzung in Gegenwart eines Komplexbildners bewirkt die gleichzeitige Komplexierung des natürlicherweise im Transferrin enthaltenen Eisens, das im Zuge der Nachreinigung, z.B. durch Ultrafiltration ausgewaschen werden kann.

Es hat sich als günstig erwiesen, die Pasteurisierung vor dem letzten Reinigungsschritt in das Herstellungsverfahren einzubauen. In diesem Stadium der Aufarbeitung besitzt das Transferrin bereits einen genügend hohen Reinheitsgrad, und diese Arbeitsweise hat den Vorteil, daß die während des Erhitzungsprozesses meist denaturierten Restmengen an Protein-Verunreinigungen durch die nachfolgende Adsorption beispielsweise an Aluminiumhydroxid leichter abzutrennen sind. Geringe Anteile an aggregiertem Transferrin werden durch die Adsorption ebenfalls entfernt.

In Kombination mit für Transferrin bekannten Reinigungsverfahren kann aus Cohn-Fraktion IV nach dem beschriebenen Verfahren pasteurisiertes, eisenfreies Human-Transferrin mit einem immunchemischen Reinheitsgrad von mindestens 98 % erhalten werden. Die erhaltene Lösung des pasteurisierten, eisenfreien Transferrins kann auf bekannte Weise nachgereinigt und konzentriert, sterilfiltriert und lyophilisiert werden. Der Anteil an Aggregaten (Dimere) liegt mit etwa 2-5 % in der gleichen Größenordnung wie der eines lyophilisierten, nichtpasteurisierten Produktes.

Das Eisenbindungsvermögen des pasteurisierten Transferrins ist voll erhalten. Eisen-freies und eisen-gesättigtes Transferrin kristallisieren nach dem Pasteurisierungsprozeß mit Hilfe von Äthylalkohol oder Polyäthylenglykol mit der gleichen Leichtigkeit und in den gleichen Kristallformen wie nicht-pasteurisiertes Transferrin. Schließlich ist die Eigenschaft des pasteurisierten Transferrins als Wachstumsfaktor in Zellkulturen zu wirken ebenfalls voll erhalten.

### Beispiel 1:

1000 g Trockenmaterial eines nach bekannten Methoden vorgereinigten Roh-Transferrins mit einem immunologischen Reinheitsgrad von etwa 90% und einem Eisen-Gehalt von 550 »g/g Protein werden in dest. Wasser gelöst und mit soviel Trinatriumcitrat versetzt, daß 20 l einer ca. 5 %igen, 1,2 M Trinatriumcitrat enthaltenden Protein-Lösung entstehen. Der pH-Wert der Lösung wird mit 1 M Citronensäure auf 7,5 eingestellt.

Die Roh-Transferrin-Lösung wird in einem Wasserbad unter ständigem Rühren auf 60°C erhitzt und 10 Stunden bei 60°C gehalten. Nach Abkühlen auf Raumtemeratur wird eine während das Erhitzungsprozesses entstandene leichte Trübung durch Klärfiltration abgetrennt.

Durch Ultrafiltration (Membranausschlußgrenze = 10 KD) wird das Natriumcitrat und das daran gebundene Eisen mit dest. Wasser aus der Protein-Lösung ausgewaschen. Der Eisen-Gehalt der Transferrin-Lösung beträgt jetzt weniger als 20 »g/g Protein.

Die sich nun anschließende Hochreinigung des eisenfreien Transferrins erfolgt mit Hilfe bekannter Methoden wie Adsorption der Restverunreinigungen an Aluminiumhydroxid oder durch Chromatographie an Anionenaustauschern.

### Beispiel 2:

Man verfährt zunächst wie in Beispiel 1; verwendet jedoch anstelle von Trinatriumcitrat Ethylendinitrilotetra-essigsäure-dinatrium-Salz in einer Konzentration von 0,5 M.

Der pH-Wert der Lösung wird mit 1 N Natronlauge auf pH 7,5 eingestellt. Die weitere Behandlung erfolgt wie in Beispiel 1 beschrieben.

## Patentansprüche (Patentansprüche für folgende(n) Vertragsstaat(en): AT, BE, CH, DE, DK, FR, GB, IT, LI, LU, NL, SE)

1. Verfahren zur Aufbereitung von Human-Transferrin, dadurch gekennzeichnet, daß eine Lösung von Human-Transferrin, die einen Komplexbildner enthält, pasteurisiert wird.

2. Verfahren nach Anspruch 1, dadurch gekennzeichnet, daß als Komplexbildner ein lösliches Citrat oder ein Salz der Ethylendiamin -tetraessigsäure (EDTA) verwendet wird.

3. Verfahren nach Anspruch 1, dadurch gekennzeichnet, daß der Komplexbildner mit dem aus dem Transferrin gebundenen Eisen abgetrennt wird.

## Patentansprüche (Patentansprüche für folgende(n) Vertragsstaat(en): ES, GR)

1. Verfahren zur Aufbereitung von Human-Transferrin, dadurch gekennzeichnet, daß eine Lösung von Human-Transferrin, die einen Komplexbildner enthält, pasteurisiert wird.

2. Verfahren nach Anspruch 1, dadurch gekennzeichnet, daß als Komplexbildner ein lösliches Citrat oder ein Salz der Ethylendiamino-tetraessigsäure (EDTA) verwendet wird.

3. Verfahren nach Anspruch 1, dadurch gekennzeichnet, daß der Komplexbildner mit dem aus dem Transferrin gebundenen Eisen abgetrennt wird.

## Claims (Claims for the following Contracting State(s): AT, BE, CH, DE, DK, FR, GB, IT, LI, LU, NL, SE)

1. A process for the treatment of human transferrin, which comprises pasteurizing a solution of human transferrin which contains a complexing agent.

2. The process as claimed in claim 1, wherein a soluble citrate or a salt of ethylenediaminetetraacetic acid (EDTA) is used as complexing agent.

3. The process as claimed in claim 1, wherein the complexing agent is removed with the bound iron from the transferrin.

## Claims (Claims for the following Contracting State(s): ES, GR)

1. A process for the treatment of human transferrin, which comprises pasteurizing a solution of human transferrin which contains a complexing agent.

2. The process as claimed in claim 1, wherein a soluble citrate or a salt of ethylenediaminetetraacetic acid (EDTA) is used as complexing agent.

3. The process as claimed in claim 1, wherein the complexing agent is removed with the bound iron from the transferrin.

## Revendications (Revendications pour l'(les) Etat(s) contractant(s) suivant(s): AT, BE, CH, DE, DK, FR, GB, IT, LI, LU, NL, SE)

1. Procédé pour la production de transferrine humaine, caractérisé en ce que l'on pasteurise une solution de transferrine humaine qui contient un agent complexant.

2. Procédé selon la revendication 1, caractérisé en ce que, comme agent complexant, on utilise un citrate soluble ou un sel de l'acide éthylènediaminetétraacétique (EDTA).

3. Procédé selon la revendication 1, caractérisé en ce que l'agent complexant est séparé avec le fer lié provenant de la transferrine.

## Revendications (Revendications pour l'(les) Etat(s) contractant(s) suivant(s): ES, GR)

1. Procédé pour la production de transferrine humaine, caractérisé en ce que l'on pasteurise une solution de transferrine humaine qui contient un agent complexant.

2. Procédé selon la revendication 1, caractérisé en ce que, comme agent complexant, on utilise un citrate soluble ou un sel de l'acide éthylènediaminetétraacétique (EDTA).

3. Procédé selon la revendication 1, caractérisé en ce que l'agent complexant est séparé avec le fer lié provenant de la transferrine.
